Europäisches Patentamt

European Patent Office

Office européen des brevets

(11) Publication number: **0 331 392**
**A2**

## EUROPEAN PATENT APPLICATION

(21) Application number: 89301916.6

(22) Date of filing: 27.02.89

(51) Int. Cl.⁴ **A61L 15/03**

(30) Priority: 01.03.88 US 162761

(43) Date of publication of application:
06.09.89 Bulletin 89/36

(84) Designated Contracting States:
AT BE CH DE ES FR GB GR IT LI LU NL SE

(71) Applicant: **ALZA CORPORATION**
**950 Page Mill Road P.O. Box 10950**
**Palo Alto California 94303-0802(US)**

(72) Inventor: **Gale, Robert M.**
**1276 Russell Avenue**
**Los Altos California 94022(US)**
Inventor: **Chin, Ivan**
**2808 Wemberly Drive**
**Belmont California 94002(US)**
Inventor: **Libicki, Shari B.**
**751-B Loma Verde Drive**
**Palo Alto California 94303(US)**

(74) Representative: **Evans, David Charles et al**
**F.J. CLEVELAND & COMPANY 40-43,**
**Chancery Lane**
**London, WC2A 1JQ(GB)**

(54) **Anesthesia and antisepsis of the skin.**

(57) A device which occludes an area of skin has a self adhering matrix containing a topical anesthetic of the amide or ester type. A backing of polymer cloth, adhered to the matrix, drapes and stretches to conform to the wearer's movements. An aggressive adhesive forms the matrix and maintains the anesthetic in intimate contact with the skin. The anesthetic agents exhibit high activity against anaerobes as well as aerobes, when occluded on the skin.

EP 0 331 392 A2

## ANESTHESIA AND ANTISEPSIS OF THE SKIN

This invention relates to anesthesia and antisepsis of the skin. More particularly this invention relates to these actions as they relate to bandages, patches, and transdermal delivery devices. Still more particularly, but without limitation thereto, this invention relates to a transdermal device for the delivery of an anesthetic to alleviate pain and as a pretreatment for a patient about to undergo a painful procedure.

The transdermal route of parental delivery of drugs provides many advantages and transdermal systems for delivering a wide variety of drugs or other beneficial agents are described in U.S. Patent Numbers 3,598,122; 3598,123; 4,286,592; 4,314,592; 4,379,454 and 4,568,343 for example, all of which are incorporated herein by reference.

Even with the variety of transdermal devices known to the art, there is a continuing need for the development of devices designed to deliver specific drugs in specific regimens. For example, devices suitable for the delivery of systemically active drugs may not provide therapeutic delivery for topical drugs and devices having a rate controlling mechanism may be especially suited for the delivery of some drugs and not suited for others.

Additionally, there is a constant need to improve upon state of the art devices to overcome any of their shortcomings. For example, some devices have components which tend to cause a burning or tingling sensation when applied to the skin and some devices tend to cause discomfort to wearers due to microbial growth that may occur on the skin under occlusion.

Further, many transdermal devices cannot or should not be applied to broken, damaged or compromised skin. Therefore, there is a need for devices which can be placed on skin that has cuts or abrasions, for the purpose of alleviating pain.

An object of this invention is to provide for the effective delivery of topical drugs, specifically anesthetics, and further to provide anesthesia by means of a thin, comfortable, unobtrusive, flexible device.

Another object of this invention is to incorporate an anesthetic into a transdermal delivery device to alleviate any tingling or burning sensation caused by application of the device, so that the device will be more comfortable to wear over extended time periods and further to provide anesthesia as a pretreatment for painful procedures.

A further object of this invention is to provide a transdermal delivery device which will deliver a pulse of an anesthetic, providing rapid delivery of anesthetic, and thus attaining rapid onset of anesthesia.

According to the present invention, there is provided a delivery device for application to a body surface or membrane to deliver an anesthetic having antimicrobial properties by permeation through said body surface or membrane, comprising:

a self adhering matrix having a topical anesthetic dispersed therethrough; and

a backing member positioned behind the skin distal surface of said matrix.

In this way, it is possible to incorporate an anesthetic into a transdermal delivery device to provide antimicrobial activity under occlusion, without increasing the obtrusiveness or bulk of the delivery device.

The devices of the invention enable a transdermal device to be placed upon broken or damaged skin for the purpose of alleviating pain.

This invention provides a device which covers an area of skin, and delivers an agent which acts both as an anesthetic and as a microbicide. The agent has a hydrophilic group which is separated from a hydrophobic group by an ester or an amide linkage.

In accordance with one embodiment of the invention, the hydrophilic group is an amine, usually secondary or tertiary substituted. The hydrophobic group is an aromatic residue. The hydrophilic and hydrophobic groups are linked by an amide as in amide type anesthetics or by an ester as in ester type anesthetics.

Typical examples include: tetracaine base or tetracaine HCL, an ester type anesthetic, which has an aromatic residue and a tertiary amine separated by an ester; and lidocaine, an amide type anesthetic, which has an aromatic residue and a tertiary amine separated by an amide. Other suitable agents include, without limitation, benzocaine, etidocaine, procaine, prilocaine, chloroprocaine and bupivacaine. As used herein, the term "anesthetic agent" refers to the agent itself, along with its base and salt form.

Following is a description by way of example only of methods of carrying the invention into effect.

In the drawings:

Figure 1 is a schematic cross-sectional view of one embodiment of this invention.

Figure 2 is a schematic cross-sectional view of another embodiment of this invention.

Figure 3 is a perspective view of the device of Figure 1 applied to an arm; and

Figure 4 is a perspective view of the device of Figure 1, illustrating its flexibility.

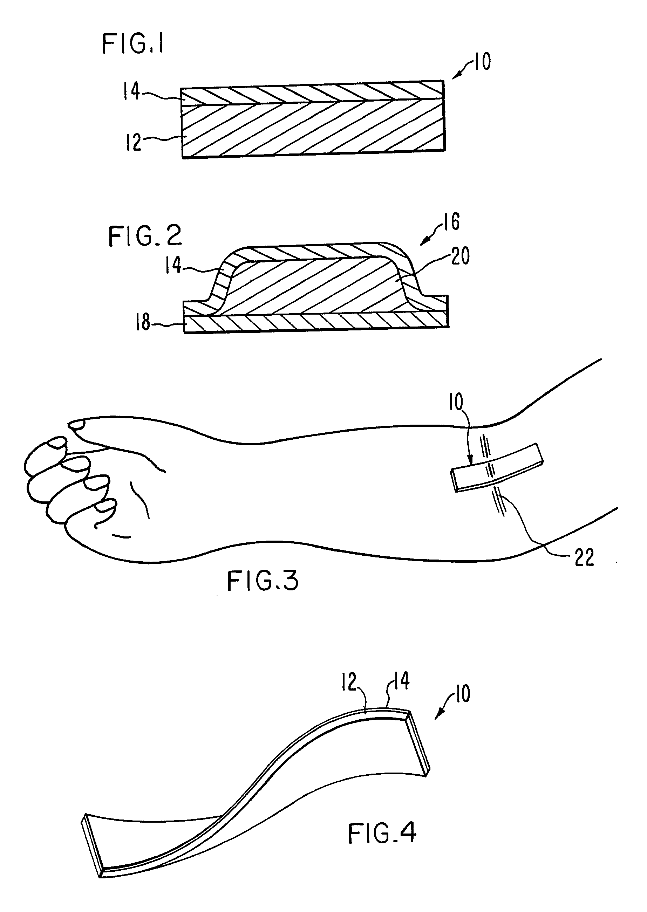

The invention is best understood with reference to the accompanying Figures. The invention shown in Figure 1 provides for a transdermal drug delivery device 10 containing an anesthetic which simultaneously serves as an antimicrobial agent. The device provides for close and continuous contact between the self adhering polymer matrix 12 and the administration site. A backing member, lamina 14 is flexible so as to conform to the wearer's movements.

Self adhering polymer matrix 12 contains a drug incorporated therein, which has anesthetic properties, and is microbicidal when in contact with the skin under occlusion. In accordance with the invention, it has been found that many topical anesthetics, particularly the "caines," possess antimicrobial properties, in an occluding environment, and act against anaerobes as well as aerobes. Particularly, tetracaine has been found quite effective. Procaine and lidocaine additionally possess anesthetic and antimicrobial properties. Either the salt form or the base form of the agent may be used, however the base tends to transport more quickly through the skin. Despite rapid transport of the base, antimicrobial activity remains high. Accordingly, the base form is preferred for maximum anesthetic effect, although a mixture of base and salt may be provided.

It is of particular importance in the promotion of anesthesia and the elimination of microorganisms to maintain excellent contact between the active agent and the skin. In accordance with the invention, polymer matrix 12 self adheres to the wearer's skin. Important criteria for the matrix material are aggressiveness, cohesion, bio-and chemical-compatability, rapid drug mass transport ability, and flexibility. Examples of suitable matrix materials include poly(styrene-butadiene) and poly(styrene-isoprene-styrene) block copolymers, and high and low molecular weight polyisobutylene copolymers. The matrix may also be of an ethylene vinyl acetate (EVA) copolymer of the type described in U.S. Pat.No. 4,144,317, preferably those having a vinyl acetate content in the range of about 28 to 60 wt%. Particularly good results have been obtained using EVA having 40 % vinyl acetate.

Adhesive properties are enhanced by adding a resinous tackifier. This is especially important when using a non-tacky polymeric matrix. Examples of suitable tackifiers include: STAYBELITE ESTER #5 or #10, REGALREZ and PICCOTAC (all of which are trademarks of Hercules of New Jersey). Additionally, the matrix may also contain a rheological agent, suitable examples of which include, without limitation, mineral oil and silica.

A dye for marking the skin may further be mixed into the matrix, or provided in a separate layer within the device. This marker die, for example gentian violet, can be used to facilitate visualization of the treated area. Alternatively, it has been found that with particular agents, at particular concentrations, mild erythema results. This condition serves to adequately mark the area, and is preferred where it is desired to avoid the need for washing or scrubbing the treated region. Alternatively, a flourescent marker may be employed.

Matrix 12 advantageously contains a plurality of drugs or substances. Along with the tackifier, rheological agent, dye and other components as are known to the art, matrix 12 may also contain an additional drug to be delivered along with the anesthetic. Additionally, the matrix may contain a permeation enhancer to facilitate the transdermal delivery of the anesthetic agent. Suitable permeation enhancers include, without limitation, glycerol monooleate and ethanol.

Backing member 14 is preferably made of a woven or non-woven cloth-like material, capable of maintaining matrix 12 in close contact with the skin. Preferred materials include non-woven polymer cloths such as rayon polypropylene or other polyester fabrics, or polyethylene or polyurethane coated spun-bonded polyester cloths. These materials have been found to conform advantageously to a wearer's movements, by draping and stretching. Moreover, the cloths are easy to handle, and are non- intimidating to the user. Specifically, the materials are commonly found in clothing and other items with which one comes into daily contact. For this reason patients, especially children, may be more receptive to the use of this type of material, as opposed to the plastic-like, flesh colored backings commonly used in transdermal devices of the prior art. Moreover, the devices may be cut to form amusing shapes.

Alternately, backing member 14 can be fabricated from a material impermeable to matrix 12 and thus form an occlusive backing. Examples of other materials which may also be used include polypropylene, polyester, polycarbonate, polyurethane or other polymer films. These films can be metalized.

Critical in the selection of the backing material is its flexibility. For this reason, an ideal candidate is one way stretchable polyester cloth, which is an anisotropic material having the stretch direction oriented perpendicular to the roll length. This fabric is approximately 0.3 mm thick and has a modulus in one direction approximately 500 times that of the modulus in the orthogonal direction. The stress-strain curve of the fabric when tested in the stretchy direction is linear up to 25 % elongation. At 25 % elongation, the stress was 24-32 $gm/mm^2$, which is an indication of the low force needed to distend the fabric. Optimum stress values at 25 % elongation lie in the range of 1-300 gm/mm² but values of greater then 100 gm/mm² are preferred. The use of fabrics which have good "drape" characteristics and which stretch at least in one direction allow patches to be applied onto body sites that are very mobile and are subjected to a large amount of bending, such as the antecubital fossa. This is an important area of the body in which to induce anesthesia because this is the usual site from which blood is withdrawn.

In a preferred embodiment, the matrix is comprised of an anesthetic (preferably tetracaine)-polyisobutylene/mineral oil and the backing is spun-bonded polyester cloth (polyurethane coated). Another embodiment of this invention is comprised typically of an anesthetic/EVA 40/STAYBELITE ESTER matrix with a SCOTCHPAK (3M Company) backing.

This invention also contemplates codelivery of a drug or therapeutic agent with the anesthetic. Preferably, the drug and anesthetic agent are contained in separate reservoirs, as is shown in Figure 2. However, this invention also contemplates placing the drug and anesthetic in a single reservoir as in Figure 1.

The invention can be configured in a variety of ways, according to the drug and a preferred delivery profile therefore. In Figure 2, system 16 is shown having a rate controlling matrix 18 comprising a microporous or other rate controlling material, an adhesive, and the anesthetic agent. A typical example of suitable materials would be: microporous polypropylene, a styrene copolymer and tetracaine, respectively. If rate control is not desired, the matrix 18 can be simply comprised of an adhesive material and the anesthetic agent.

Drug matrix 20 contains a drug to be codelivered with the agents within rate controlling layer 18, and may also contain anesthetic agent. The materials listed herein as being suitable for use as the self adhering matrix are also suitable for use as the drug matrix.

The rate controlling matrix serves to control the delivery rate of drug from matrix 20. The rate controlling matrix is such that the in vitro drug flux of drug through it is less than the in vitro flux of drug across the skin. However, this invention also contemplates use of a rate controlling matrix having an in vitro flux greater than or equal to the rate of drug flux across the skin.

The device may be provided in appropriate sizes, typical surface areas being from 1 to 100 cm squared. An oversized removable liner, not shown, facilitates packaging and use. A preferred material for the removable liner is silicone coated paper.

Figure 3 illustrates the draping effect of device 10 in accordance with the invention. Device 10 is longitudinally shaped to overlap an area of skin over a large vein. In this position, the device is useful as a pretreatment for venipuncture or other painful procedure. In Fig. 3, the arm is substantially outstretched. Note that the device 10 remains well adhered to the skin, even at the skin fold 22. This adhesion is maintained before and after the arm is folded. It should be understood, however, that a device in accordance with the invention can be die cut to a wide variety of shapes and sizes appropriate for the intended use.

A device in accordance with this invention can be manufactured by numerous state of the art methods, including extruding or solvent casting the matrix upon a continuous web of the backing material. In one embodiment, a removable liner is laminated over the matrix, or the matrix is first laminated onto the removable liner. Devices are die cut from the web in appropriate sizes. The device is used by peeling off the removable liner and applying the matrix and backing to the skin.

Specific examples of devices, systems, or structures are detailed below. Generally, the device contains a polymer matrix, a resinous tackifier, a rheological agent, an antioxidant and the active anesthetic agent. The proportions of each are interdependent.

A preferred embodiment of the self adhering matrix of this invention is comprised of the following components on a weight percent basis: 10-70 % preferably 15-50 % polymer matrix material; 20-70 %, preferably 30-60 % tackifier; 0-30 % preferably 7-25 % rheological agent; 0-5 % preferably 0.4-2 % antioxidant; and 0.1-20 % preferably 5-15 % anesthetic.

In some instances, anesthetics may sensitize or irritate the skin. Therefore, it may be desirable to incorporate an agent which will prevent adverse reactions between the anesthetic and the skin. It has been found that phenyl alkyl alcohols such as phenyl ethyl alcohol incorporated into a tetracaine system, will prevent any of the adverse skin reactions that can occur with tetracaine delivery. Such an agent or sensitization inhibitor can be incorporated into the system in an amount within the range of 1-20 weight percent, preferably 5-15 weight percent. A specific example using such an agent and having a composition within the ranges set forth above, is provided in Example I.

EXAMPLE I

4

In order to evaluate the release rates and onset of anesthesia, tetracaine patches were made according to this invention by blending by weight percent: 20.3 % poly(styrene-isoprene-styrene) block copolymer (KRATON 1112 Rubber, Shell Oil Company), 51.0 % tackifier (REGAL-REZ 1094, Hercules), 13.6 % mineral oil, 0.7 % antioxidant (IRGONOX 1010, Ciba-Geigy Corporation), 7.0 % phenyl ethyl alcohol and 7.4 % tetracaine base, in an internal mixing bowl at 100° C.

After approximately one hour of blending, the mixture was extruded between two release substrates. One substrate was peeled away and a stretchable polyester backing was applied in its place. Individual patches were die cut for subsequent in vitro and in vivo testing. The range of drug loading was varied between 0.1 and 1.5 mg/cm$^2$ for a film thickness of 0.004 inches.

The in vitro drug release rate of these patches increased in magnitude with increased drug loading. The in vitro drug flux across cadaver epidermis also increased from 20 $\mu$g/hr/cm$^2$ at the lowest drug loading to more than 100 $\mu$g/hr/cm$^2$ at the greatest drug loading. It was also found that the duration of steady state drug skin flux varied from less than one hour to greater than four hours as the drug loading was increased.

When these patches were placed upon the intact skin of human subjects, local anesthesia was induced under the application site. The degree and duration of the block, as well as the onset time, depended upon the drug loading. When the drug loading was within the range of 0.1-0.6 mg/cm$^2$, very little effect was found even after 2-3 hours application time. When the loading was increased to 1.0 mg/cm$^2$, the quality of the anesthetic block was better but the extent of the block was widely varied among the test panel. When the loading was increased to 1.5 mg/cm$^2$ and greater, the anesthetic block achieved was excellent among all subjects. The onset time to achieve the block varied between 40 minutes to 1.5 hours and the duration of the block was within the range of 3-6 hours. Therefore, this invention contemplates using a drug loading of at least 1.0 mg/cm$^2$, preferably at least 1.5 mg/cm$^2$.

## EXAMPLE II

A self-adhesive transdermal delivery device in accordance with the invention could be fabricated by extrusion. The following ingredients would be combined on a rubber mill until well mixed:

| | |
|---|---|
| Polyisobutylene mw 1200k | 26.2 wt % |
| Polyisobutylene mw 35 k | 39.3 wt % |
| Light Mineral Oil | 28.0 wt % |
| Gentian Violet | 0.5 wt % |
| Tetracaine | 6.0 wt % |

The mixture would then be remelted in an extruder screw feeder, and extruded to a thickness of about 75 micrometers ($\mu$m) onto a web of 200 $\mu$m porous rayon polypropylene cloth. The web could then be die-cut into strips 2 by 6 cm.

This invention provides for extremely rapid anesthesia. By incorporating the anesthetic within an adhesive base, the agent is maintained in intimate contact with the skin over the entire occluded surface area. Significant anesthesia has been observed with the matrix described in Example II in as little as 35 minutes. Moreover, the adhesive can be formulated to be quite aggressive, yet is not uncomfortable to remove since the occluded area is anesthetized. Additionally, devices in accordance with the invention do not leave a cream residue on the skin after the system is removed.

## EXAMPLE III

A self-adhesive transdermal delivery device in accordance with the invention can also be fabricated by solvent casting. The following ingredients would be stirred until well mixed:

| Polyisobutylene mw 1200k | 5.3 wt % |
|---|---|
| Polyisobutylene mw 35 k | 7.9 wt % |
| Light Mineral Oil | 5.6 wt % |
| Tetracaine | 1.2 wt % |
| Hexane | 80.0 wt % |

The resultant mixture would be cast on a web to a wet thickness of about 750 $\mu$m. The solvent would then be evaporated, yielding a web having a final thickness of about 75 $\mu$m.

One of the problems associated with transdermal delivery of drugs under occlusion is that the area of skin occluded tends to be the site of microbial activity, the result of which can cause a great deal of discomfort to the wearer. Therefore, a distinct advantage of this invention is that the anesthetic agents disclosed herein, also exhibit antiseptic or antimicrobial properties, as is shown by the following example.

## EXAMPLE IV

In order to illustrate the antimicrobial effect of anesthetics, monoliths containing tetracaine were compared with both monolithic patches containing PVP-I, a GAF Corporation brand of povidone-iodine, a topical anti-infective, and with placebo or control systems.

A tetracaine monolith was prepared by mixing 31.8 wt % ethylene vinylacetate having 40 wt % vinylacetate content (EVA 40), 22.2 wt % glycerol monooleate (GMO) flux enhancer to promote passage of drug through the skin, 27.8 wt % tetracaine base and 18.2 wt % STAYBELITE ESTER #5. The backing used was SCOTCHPAK (3M Company), a bonded laminate containing EVA, polyester, aluminum and polyethylene.

Three PVP-iodine (PVP-I) monoliths were prepared using varying amounts of the agent. The matrices were: 1 wt % PVP-I and 99 wt% EVA 40; 5 wt % PVP-I and 95 wt % EVA 40; and 10 wt % PVP-I and 90 wt % EVA 40. All three monolithic patches used SCOTCHPAK backing.

Two control systems were prepared. System A was comprised of 25 wt % GMO, 47 wt % EVA 40 and 38 wt % STAYBELITE ESTER. System B was comprised of 63.6 wt % EVA 40 and 36.4 wt % STAYBELITE ESTER. Both systems had SCOTCHPAK backings.

The monoliths were innoculated with S. aureus ATCC 6538, a human skin pathogen, by application of washed cells to the patch surface. The innoculated systems were incubated at 30-35° C in 75% RH. At the end of the designated time intervals, the patch surfaces were swabbed. Quantitative results were obtained by the pour plate technique on TSA media and are presented in the following table.

### TABLE I

| Inocula Concentration ( S.aureus) ATCC 6538) | | | | | |
|---|---|---|---|---|---|
| 3.8 x 10$^4$ CFU/system (active systems) | | | | | |
| 6.2 x 10$^4$ CFU/system (placebo systems) | | | | | |
| Microbial Recovery (CFU/System/10 ml Extracts) | | | | | |
| | Active Systems | | | | Controls |
| Time, min. | Monolith/Tetracaine | Monolith/1% PVP-I | Monolith/5% PVP-I | Monolith/10% PVP-I | System A | System B |
| 0 | not done | not done | not done | not done | 5.8x10$^4$ | 5.5x10$^4$ |
| 15 | 5.5x10$^3$ | 1.4x10$^4$ | 2.4x10$^3$ | 3.4x10$^3$ | 6.8x10$^4$ | 6.5x10$^4$ |
| 30 | -0- | 3.6x10$^2$ | -0- | 1.6x10$^2$ | 6.1x10$^4$ | 5.0x10$^4$ |
| 60 | -0- | 3.0x10$^1$ | -0- | -0- | 2.8x10$^4$ | 6.0x10$^4$ |

Table II illustrates, in vivo, the antimicrobial effects of the tetracaine containing monolith of Fig. 1. Indigenous fauna in the region adjacent an occluding device was determined as the number of colonies on TSA and EUG pour plates. These figures are compared with results obtained by pour plating swabs

6

obtained in an identical manner from a region occluded 30 minutes, for six human individuals tested.

TABLE II

| | Subject | Non-Occluded | Occluded |
|---|---|---|---|
| AEROBIC BACTERIAL CONTENT (CFU/ 7.0 cm$^2$ | A | $5.0 \times 10^3$ | $1.5 \times 10^2$ |
| | B | $7.8 \times 10^4$ | $2.5 \times 10^2$ |
| | C | $5.0 \times 10^1$ | 0 |
| | D | $1.0 \times 10^2$ | 0 |
| | E | $2.3 \times 10^4$ | 0 |
| | F | $5.0 \times 10^1$ | 0 |
| | Subject | Non-Occluded | Occluded |
| ANAEROBIC BACTERIAL CONTENT | A | $1.5 \times 10^4$ | $5.5 \times 10^2$ |
| | B | $7.5 \times 10^2$ | $1.0 \times 10^2$ |
| | C | $1.5 \times 10^2$ | $5.0 \times 10^1$ |
| | D | $6.0 \times 10^4$ | $7.5 \times 10^3$ |
| | E | $8.3 \times 10^4$ | $4.0 \times 10^2$ |
| | F | $1.0 \times 10^2$ | 0 |

Experiments conducted by the inventors have revealed that common antimicrobial agents do not work well against all microorganisms in occluding systems, such as transdermal drug delivery devices. The following materials were tested, under an occluding skin patch, for antimicrobial activity: chlorhexidine gluconate, methyl paraben, propyl paraben, GERMALL 115 (a trademark of Sutton Labs of New Jersey for an amidazolidinyl urea), benzethonium chloride, and phenylmercuric acetate. Experimental and control systems comprised an impermeable backing and adhesive. Experimental systems additionally contained the antimicrobial agent. Organism numbers were determined by pour plating on aerobe, fungal, and anaerobe growth media. Results showed that good to excellent kill rates were obtained for aerobes, while anaerobe numbers were moderately reduced with the use of any of the tested materials. It is suggested that typical antimicrobial agents such as these are impaired by sebum and lecithin, common materials found in the skin and in hair follicles, where microorganisms proliferate. Thus, a distinct advantage of this invention is that the anesthetics disclosed herein also perform as antimicrobial agents as is evidenced by the above-noted tables.

Table III illustrates results of an additional in vivo experiment. Microbial growth was sampled under a matrix as described in Example II, without gentian violet, and a placebo as in Example II, both without gentian violet and without tetracaine. Background colony counts for the active and placebo systems were tested before application, and at the indicated intervals. Microbial growth was indicated by colonies on BAP, EUG and MYC media.

## TABLE III

### SUBJECT #1

| TIME | ACTIVE SYSTEMS | | | PLACEBO SYSTEMS | | |
|------|------|------|------|------|------|------|
| (HR) | BAP | EUG | MYC | BAP | EUG | MYC |
| 0 | 20 | $1.7\times10^3$ | 0 | 260 | $1.4\times10^3$ | 0 |
| 1 | $1.2\times10^2$ | $1.7\times10^3$ | 0 | 40 | $7.0\times10^2$ | 0 |
| 1.5 | 0 | 0 | 0 | 40 | $2.4\times10^3$ | 20 |
| 2.0 | 0 | 0 | 0 | 0 | $1.3\times10^3$ | 0 |
| 3.0 | 0 | 0 | 0 | 20 | $3.0\times10^2$ | 0 |
| 5.0 | 0 | 0 | 0 | 80 | $3.4\times10^2$ | 0 |

## TABLE III, con't

### SUBJECT #2

| TIME | ACTIVE SYSTEMS | | | PLACEBO SYSTEMS | | |
|------|------|------|------|------|------|------|
| (HR) | BAP | EUG | MYC | BAP | EUG | MYC |
| 0 | 20 | $1.2\times10^2$ | 0 | $2.4\times10^2$ | $1.4\times10^3$ | 0 |
| 1 | 20 | $1.6\times10^2$ | 0 | $1.4\times10^2$ | $1.5\times10^4$ | 0 |
| 1.5 | 40 | 40 | 0 | 20 | $2.8\times10^2$ | 0 |
| 2.0 | 40 | 0 | 0 | 80 | $3.0\times10^2$ | 0 |
| 3.0 | 0 | 0 | 0 | 40 | $2.8\times10^2$ | 0 |
| 5.0 | 0 | 0 | 0 | 40 | 80 | 0 |

Additionally, the delivery device of this invention can be used to deliver a therapeutic agent and at the same time anesthetize the skin. This is illustrated by the following example.

## EXAMPLE V

A transdermal delivery device for the regulation of blood pressure can be constructed according to embodiment of Fig.2, whereby clonidine and tetracaine are delivered. Clonidine has been known to sensitize some patients and therefore inclusion of tetracaine in the system will help to alleviate any discomfort. The drug matrix would be comprised of a mixture of high and low molecular weight polyisobutylene, clonidine, mineral oil and colloidial silicon dioxide. A standard SCOTCHPAK backing would be suitable. The rate controlling matrix would contain the same materials as the drug matrix. Clonidine present in the rate controlling matrix would be readily available when the system was placed on the skin and thus would provide a priming dose. Additionally, the rate controlling matrix would contain tetracaine base or tetracaine HCl and microporous polypropylene, the latter of which provides the rate controlling

8

properties.

While various aspects of the invention have been set forth by the drawings and the specification, it is to be understood that the foregoing detailed description is for illustration only and that various changes in parts, as well as the substitution of equivalent constitutes for those shown and described, may be made without departing from the spirit and scope of the invention as set forth in the appended claims.

**Claims**

1. A delivery device for application to a body surface or membrane to deliver an anesthetic having antimicrobial properties by permeation through said body surface or membrane, comprising:
a self adhering matrix having a topical anesthetic dispersed therethrough; and
a backing member positioned behind the skin distal surface of said matrix.

2. A device as claimed in claim 1 wherein said topical anesthetic is selected from amide type anethetics and ester type anesthetics.

3. A device as claimed in claim 2 wherein said topical anesthetic is selected from tetracaine, lidocaine, benzocaine, etidocaine, procaine, prilocaine, dibucaine, chloroprocaine and bupivacaine.

4. A device as claimed in any preceding claim wherein said self adhering matrix is comprised of an adhesive material selected from poly(styrene butadiene) and poly(styrene-isoprene-styrene) block copolymers, high and low molecular weight polyisobutylene copolymers and ethylene vinyl acetate copolymers.

5. A device as claimed in any preceding claim wherein said backing member is a material selected from polyester fabrics, polyethylene coated spun-bonded polyester cloth, polyurethane coated spun-bonded polyester cloth, rayon polypropylene, polyprop[ylene, polyester, polycarbonate and polyurethane.

6. A device as claimed in any preceding claim wherein said self adhering matrix comprises 15-50 wt% adhesive material; 30-60 wt% tackifier; 7-25 wt% rheological agent; 0.4-2 wt% antioxidant; and 5-15 wt% anesthetic.

7. A device as claimed in claim 6 wherein said self adhering matrix further comprises about 5-15 wt% sensitization inhibitor.

8. A device as claimed in claim 7 wherein said adhesive material is poly(styrene-isoprene-styrene) block copolymer, said rheological agent is mineral oil, said anesthetic is tetracaine base and said sensitization inhibitor is phenyl ethyl alcohol.

9. A device as claimed in any preceding claim wherein the drug loading of said anesthetic is at least ·1.0 mg/cm$^2$.

10. A device as claimed in claim 9 wherein the drug loading of said anesthetic is at least 1.5 mg/cm$^2$.

FIG.1

14

12

10

FIG.2

14

20

18

16

10

22

FIG.3

12

14

10

FIG.4